# EUROPEAN PATENT SPECIFICATION

(11) **EP 3 025 705 B1**
(45) Date of publication and mention of the grant of the patent: **24.10.2018**
(21) Application number: 14194774.7
(22) Date of filing: 25.11.2014
(51) Int. Cl.: A61K 9/16

(54) **Stable ivabradine formulations**
Stabile Ivabradinformulierungen
Formulations d'ivabradine stable

(43) Date of publication of application: 01.06.2016
(73) Proprietor: Zentiva Saglik Ürünleri Sanayi Ve Ticaret A.S., 39780 Kirklareli (TR)
(72) Inventor: Adiyaman, Mustafa, 39780 Kirklareli (TR); Oran, Umut, 39780 Kirklareli (TR); Cakmak, Özkan, 39780 Kirklareli (TR)
(74) Representative: Sevinç, Erkan

(56) References cited:
- US-A- 5 296 482
- US-A1- 2011 306 598
- US-A1- 2013 084 335

## Description

### Field of the Invention

The present invention relates to a stable pharmaceutical formulation of ivabradine oxalate, in particular the pharmaceutical formulation comprises ivabradine oxalate, at least one antioxidant and one or more pharmaceutically acceptable excipients; wherein said at least one antioxidant comprises butylated hydroxy toluene and ascorbic acid and said at least one antioxidant is being present in an amount of from 0,05% to 5% by weight of the total composition.

### Background of the Invention

Ivabradine, or namely 7,8-dimethoxy-3-(3-[[(1S)(4,5-dimethoxybenzocyclobutan-1-yl)methyl]-methylamino]propyl)-1,3,4,5-tetrahydro-2H-benzazepin-2-one, which is a cardiotonic agent, is a novel medication used for the symptomatic management of angina pectoris. Ivabradin acts by reducing the heart rate via specific inhibition of the funny channel.

Synthesis routes for the preparation of ivabradine and its use for preventing and treating various clinical conditions of myocardial ischaemia, supraventricular arrhythmias and coronary arteriosclerotic episodes are disclosed in EP 534 859.

Ivabradine and addition salts thereof have very valuable pharmacological and therapeutic properties, especially bradycardic properties, making those compounds useful in the treatment or prevention of various clinical situations of myocardial ischemia such as angina pectoris, myocardial infarct and associated rhythm disturbances, and also in various pathologies involving rhythm disturbances, especially supraventricular rhythm disturbances, and in heart failure.

Hydrochloride salt of ivabradine is known very well in the prior art; especially from the teaching of product PROCORALAN® which is available in the market. The preparation and therapeutic use of ivabradine hydrochloride have been described in EP 534 859.

Apart from the hydrochloride salt of ivabradine, other acid addition salts of the compound are known in the prior art. For instance, WO 2011/157720 relates to a pharmaceutical composition having modified release profile, comprising ivabradine adipate and a combination of a water-insoluble and a water-soluble excipient. The patent application EP 2 276 742 is directed to ivabradine hydrobromide. US 2013/0158008 A1 discloses the problem with the salts and polymorphs of ivabradine, in particular the polymorphs of the hydrochloride. Problems caused by the inhomogeneous distribution are also mentioned within the disclosure of US 2013/0158008 A1. The active substance ivabradine is homogeneously and molecularly disperse mixed with a pharmaceutically acceptable excipient to ensure the stability of the active substance and the homogeneous distribution in the final formulation.

An overriding problem with any pharmaceutical formulation is the stability of the active ingredient. By-product materials which are unwanted for the sake of the quality of the medicament may cause toxicity problems. The polymorphic stability is also highly desirable. Polymorphics changes may result in poor bioavailability.

Unfortunately pharmaceutical formulations comprising ivabradine hydrochloride shows polymorphic transformations during storage. This, in turn can lead to problems in processing as well as during storage and cause undesired reactions with the excipients employed in preparation of the pharmaceutical formulation. This, of course, is not acceptable and pharmaceutical formulations of ivabradine having polymorphic stability are highly desired.

Bearing in mind said problems; it is therefore an object of the present invention to provide a pharmaceutical formulation of ivabradine in which the active agent retains its polymorphic form and is therefore highly stable against conversion into other polymorphic forms.

It has been surprisingly discovered that, pharmaceutical formulations comprising ivabradine oxalate as active agent exhibit polymorphic stability upon storage, thereby ensuring an adequate shelf life of the final dosage form.

However, the stabililty has not still reached the desired level. Furthermore, a problem in the manufacture of pharmaceutical formulations comprising ivabradine oxalate is their sensitivity towards oxidative degradation and decomposition.

Oxidation, the greatest reason of the decomposition of the material, is caused when the α-carbon, which is located in the amide functional group of ivabradine, is activated and react with the oxygen in the air; thereby forming an impurity having the chemical formula *(S)*-7,8-dimethoxy-3-{3-{*N*-[(4,5-dimethoxy benzocyclobut-1-yl)-methyl]-*N*-(methylaminopropyl}-4,5-dihydro-2*H-*3-benzazepin-1,2-dione (hereinafter called "impurity F") and other unknown impurities above acceptable limits which are difficult to remove subsequently.

Ivabradine oxalate is susceptible to degradation when exposed to elevated temperatures and/or air during manufacturing processes. Unwanted impurity substances; especially impurity F and other unknown impurities, which can lessen drug effectiveness in compositions are occured above acceptable limits.

Thus there is a need for improved ivabradine oxalate compositions and methods of manufacturing such compositions in which the tendency for degradation of the ivabradine oxalate is reduced, resulting in said compositions having desired stability properties.

It was surprisingly found that pharmaceutical formulation comprising ivabradine oxalate, at least one antioxidant selected from the group consisting of butylated hydroxy toluene, butylated hydroxy anisol, ascorbic acid, citric acid, tocopherol, propyl gallate or fumaric acid, and one or more pharmaceutically acceptable excipient(s) significantly reduce the level of impurities and are stable on long term storage by preventing oxidative degradation.

The invention relates to a stable pharmaceutical formulation comprising ivabradine oxalate, at least one antioxidant selected from the group consisting of butylated hydroxy toluene, butylated hydroxy anisol, ascorbic acid, citric acid, tocopherol, propyl gallate or fumaric acid, and one or more pharmaceutically acceptable excipient(s) for oral administration. More particularly, the invention relates to a stable pharmaceutical formulation comprising ivabradine oxalate, an antioxidant mixture comporising ascorbic acid and butylated hydroxy toluene (hereinafter called "BHT") and one or more pharmaceutically acceptable excipient(s) for oral administration.

One of the objectives of the invention relates to a stable pharmaceutical formulation comprising ivabradine oxalate, an antioxidant mixture comprising ascorbic acid and BHT and one or more pharmaceutically acceptable excipient(s) for oral administration.

Another objective of the invention relates to a process of preparing a stable pharmaceutical formulation comprising ivabradine oxalate, antioxidant mixture comprising ascorbic acid and BHT and one or more pharmaceutically acceptable excipient(s).

Another objective of the invention relates to a stable pharmaceutical formulation in the form of tablet comprising ivabradine oxalate, antioxidant mixture comporising ascorbic acid and BHT and one or more pharmaceutically acceptable excipient(s).

### Detailed Description of the Invention

The invention relates to a stable pharmaceutical formulation comprising ivabradine oxalate, an antioxidant mixture comprising ascorbic acid and BHT in an amount of from 0,05% to 5% by weight of the total composition and one or more pharmaceutically acceptable excipient(s) for oral administration. As mentioned in the background of the invention, it is very difficult to prepare a stable pharmaceutical formulation of ivabradine oxalate because of its oxidative degradation and thereby increasing the level of impurities in the dosage form.

The present inventors have conducted intensive studies and surprisingly found that stability of a pharmaceutical formulation comprising ivabradine oxalate can be increased by adding the mixture of ascorbic acid and BHT; thereby a pharmaceutical formulation is provided which is stable throughout its shelf-life and is prevented mainly from oxidative degradation, with the consequence that content of impurities is maintained low.

The pharmaceutical formulations of the invention have advantages over compositions which do not contain the antioxidant mixture discussed herein. Most importantly, ivabradine oxalate preserved from oxidative attack during the storage through said antioxidant mixture.

According to an embodiment of the invention, it includes a stable pharmaceutical formulation comprising ivabradine oxalate.

According to an embodiment of the invention, it includes a stable pharmaceutical formulation comprising ivabradine oxalate, at least one antioxidant and one or more pharmaceutically acceptable excipient(s); wherein ascorbic acid and BHT are present as antioxidant in an amount of from 0,05% to 5% by weight of the total composition.

According to an embodiment of the invention, it includes a stable pharmaceutical formulation in tablet form comprising ivabradine oxalate as active agent, ascorbic acid and BHT as antioxidant and one or more pharmaceutically acceptable excipient(s); wherein the amount of "impurity F", other unknown impurities and total impurities remain substantially unchanged upon storage at 40°C and 75% relative humidity for a period of six months.

Stability problems due to degradation and oxidation has been overcome by using the pharmaceutical formulation comprising ivabradine oxalate, at least one antioxidant comprising butylated hydroxy toluene and ascorbic acid and one or more pharmaceutically acceptable excipient(s); wherein the weight ratio of antioxidant(s) to ivabradine oxalate ranges from 0,008 to 0,75, preferably 0,03 to 0,4. It has surprisingly found that the specific weight ratio of ivabradine oxalate and antioxidant(s) has superior effects on stability.

The antioxidant used in the present invention may include butylated hydroxy toluene (BHT), butylated hydroxy anisol (BHA), erythorbic acid, ascorbic acid, sodium ascorbate, calcium ascorbate, propyl gallate, octyl gallate, ascorbyl palmitate, tocopherol, alpha-tocopherol, vitamin E, biflavonoids, citric acid, anhydrous citric acid, sodium citrate, sodium citrate anhydrous, sodium pyrosulfite, sodium nitrite, sodium tatrate, potassium metabisulphite, potassium bisulphite, potassium sulphite, ethylenediamine tetraacetate, acetyl cysteine, fumaric acid, lecithin, 2,4,5-trihydroxybutyrophenone, 4-hydroxymethyl-2,6-di-tert-butylphenol, erythorbic acid, gum guaiac, thiodipropionic acid, dilauryl thiodipropionate, tert-butylhydroquinone and the combinations thereof.

It has been discovered that; when ascorbic acid and BHT are used together, enhanced stability for ivabradin oxalate is provided; thereby degradation due to oxidation of the pharmaceutical formulations containing ivabradine oxalate can be overcome. Because of this finding, the antioxidant mixture comprises ascorbic acid and BHT.

According to an embodiment of the invention, it includes a stable pharmaceutical formulation comprising ivabradine oxalate, ascorbic acid and BHT as antioxidant and one or more pharmaceutically acceptable excipient(s); wherein the weight ratio of ascorbic acid to BHT ranges from 0,05 to 20, preferably 0,1 to 10 and more preferably 0,5 to 5.

In the present invention, antioxidant is present in the stabilized ivabradine oxalate compositions in an effective amount to retard or prevent degradation of ivabradine, thereby stabilizing the composition. When the antioxidant amount is low, it is difficult to attain the expected drug stability, and when it is large in amount, safety problem may arise since it exceeds the allowable daily dose.

The present inventors have surprisingly found that using at least one antioxidant in the range of 0,05% to 5%, preferably 0,10% to 2,50% by weight of the total composition improves the stability of ivabradine oxalate formulations. It is unexpectedly seen that oxidation of the pharmaceutical formulations containing ivabradine oxalate can be overcome via the use of the antioxidant mixture in the specific amount.

The wording herein below is implied with in the concept of invention as follows:
The term "long term storage" as used herein, refers to pharmaceutical formulation of ivabradine oxalate having storage or shelf-life for at least 24 weeks at different temperature and storage conditions.

The term "impurity" as used herein, refers to ivabradine oxalate degradation product as formed in the pharmaceutical formulation upon storage at various conditions.

The term "stable pharmaceutical formulation" means that the amount of the corresponding total impurities within the pharmaceutical formulation comprising ivabradine oxalate as active agent, ascorbic acid and BHT as antioxidant and one or more pharmaceutically acceptable excipients has not increased by more than 1,05% by weight from the initial amount of ivabradine after storage at about 40°C and 75% relative humidity for 6 months period.

The term "pharmaceutical formulation" as used herein, refers to a formulation comprising ivabradine oxalate which may be in the form of granules, pellets, mini-tablets, tablets, modified release tablets and capsules filled with granules or pellets. More preferably, the pharmaceutical formulation is in a tablet dosage form.

The doses of ivabradine oxalate may be in the range of 2,5mg to 15mg, preferably 5mg to 10 mg.

The dosage form may be adapted for administration to the patient by any desired route of administration. For example, dosage forms include those adapted for oral administration such as tablets, film-coated tablets, effervescent tablets, orally disintegrating tablets, capsules, caplets, pills, lozenges, powders, syrups, elixirs, suspensions, solutions, emulsions, sachets and cachets; parenteral administration such as sterile solutions, suspensions, implants and powders for reconstitution; transdermal administration such as transdermal patches; rectal and vaginal administration such as suppositories, pessaries and foams; inhalation and intranasal administration such as dry powders, aerosols, suspensions, and solutions (sprays and drops); topical administration such as creams, ointments, lotions, solutions, pastes, drops, sprays, foams and gels; ocular such as drops, ointment, sprays, suspensions and inserts; and buccal and sublingual administration such as lozenges, patches, sprays, drops, chewing gums and tablets.

The pharmaceutical formulation of the invention in addition to the active ingredient may comprise one or more pharmaceutically acceptable excipient(s) which include, but are not limited to diluent(s), binder(s), disintegrant(s), antioxidant(s), glidant(s), lubricant(s), aqueous and non-aqueous solvent(s).

Suitable diluent(s) according to the invention include, but are not limited to lactose, lactose monohydrate, lactose anhydrous, sucrose, dextrose, maltodextrin, maize starch, potato starch, rice starch, wheat starch, potato starch, pregelatinized starch, sorbitol, manntiol, microcrystalline cellulose, silicified microcrystalline cellulose, cellulose derivatives, dicalcium phosphate, dextrates, dextrin, calcium carbonate, calcium sulfate, dibasic calcium phosphate dihydrate, tribasic calcium phosphate, magnesium carbonate or magnesium oxide and the combinations thereof.

Suitable binder(s) according to the invention include, but are not limited to maize starch, wheat starch, rice starch, potato starch etc, lactose, acacia, alginic acid, carbomer copolymer, copovidone, dextrin, gelatin, guar gum, cellulose derivatives such as microcrystalline cellulose, ethyl cellulose, hydroxypropyl cellulose, hydroxypropylmethyl cellulose, methylcellulose, maltodextrin, pregelatinized starch, polyvinylpyrrolidone, povidone, polyethylene oxide, sodium carboxymethylcellulose and the combinations thereof.

Suitable disintegrant(s) according to the invention include, but are not limited to cross-linked polyvinylpyrollidone, sodium starch glycolate, maize starch, pregelatinized starch, salts of carboxy methyl cellulose, microcrystalline cellulose, alginic acid, sodium alginate, guar gum, low-substituted hydroxypropyl cellulose and the combinations thereof.

Suitable glidant(s) and lubricant(s) according to the invention include, but are not limited to silica, colloidal silica, magnesium trisilicate, talc, stearic acid, magnesium stearate, calcium stearate, zinc stearate, sodium stearyl fumarate, glyceryl behenate and the combinations thereof.

Suitable aqueous and non-aqueous solvent(s) according to the invention include, but are not limited to purified water, isopropyl alcohol, ethanol, methylene chloride, acetone and mixtures thereof. Preferably, aqueous solvent(s) are used.

The pharmaceutical formulation according to an embodiment of the invention may optionally be coated with functional and/or non-functional coating to provide ease of swallowing and an elegant appearance. Suitable film-coating materials according to the invention include, but are not limited to hydroxypropylmethylcellulose (HPMC), hydroxypropylcellulose, acrylate-methacrylate copolymers, polyethylene glycol, polyvinylpyrrolidone, polyvinylpyrrolidone- vinyl acetate copolymer (PVP-VA), pullulan, pigments, dyes, lakes, titanium oxide, iron oxide, talc, macrogol, glycerol and the combinations thereof.

The stable ivabradine oxalate formulation according to the invention may be prepared by any suitable process, such as direct compression, wet granulation or dry granulation. For ivabradine oxalate containing tablets, granulation method is preferred, wet granulation is being most preferred.

According to an embodiment of the invention, the process for preparing stable pharmaceutical formulation comprising ivabradine oxalate, at least one antioxidant and one or more pharmaceutically acceptable excipient(s) involves the following steps: one or more pharmaceutically acceptable excipient is granulated with a granulation solution comprising ivabradin oxalate for obtaining granulates; said granulates are dried and then compressed into tablets or filled into capsules.

According to an embodiment of the invention, the process for preparing stable pharmaceutical formulation comprising ivabradine oxalate, at least one antioxidant and one or more pharmaceutically acceptable excipient(s) involves the following steps: granulating ivabradine oxalate with pharmaceutically acceptable excipients for obtaining granulates, drying the granulates then compressing into tablets or filled into capsules.

More specifically, an embodiment of the invention relates to a process for preparing a stable pharmaceutical formulation comprising ivabradine oxalate as active agent, ascorbic acid and BHT as antioxidant and one or more pharmaceutically acceptable excipient(s) involves the following steps: granulating ivabradine oxalate with pharmaceutically acceptable excipients and/or at least one antioxidant for obtaining granulates, drying said granulates then compressing into tablets, and optionally said tablets are coated.

According to an embodiment of the invention, it includes the process for preparing a stable pharmaceutical formulation comprising ivabradine oxalate as active agent, ascorbic acid and BHT as antioxidant and one or more pharmaceutically acceptable excipient(s) involves the following steps:
- mixing ivabradine oxalate, one or more pharmaceutically acceptable excipient and/or one or both of antioxidants to obtain a blend,
- obtaining a granulation solution comprising a solvent, one or more pharmaceutically acceptable excipient and/or at least one anti-oxidant,
- granulating said blend with said granulation solution,
- drying the granulates,
- compressing the granulates into tablets,
- and optionally coating said tablets.

According to an embodiment of the invention, it includes the process for preparing a stable pharmaceutical formulation comprising ivabradine oxalate as active agent, ascorbic acid and BHT as antioxidant and one or more pharmaceutically acceptable excipient(s) involves the following steps:
- mixing ivabradine oxalate, one or more pharmaceutically acceptable excipient and/or one or both of antioxidants to obtain a blend,
- obtaining a granulation solution comprising ethanol and butylated hydroxytoluen,
- granulating said blend with said granulation solution,
- drying the granulates,
- compressing the granulates into tablets,
- and optionally coating said tablets.

The inventors have surprisingly found that, when ivabradine is granulated with a granulation solution comprising ethanol and butylated hydroxytoluen; degradation is prevented from oxidatve degradation, thereby stability is improved.

According to an embodiment of the invention, it includes the process for preparing a stable pharmaceutical formulation comprising ivabradine oxalate, ascorbic acid and BHT as antioxidant and one or more pharmaceutically acceptable excipient(s) involves the following steps:
- mixing ivabradine oxalate, one or more pharmaceutically acceptable excipient and/or at least one antioxidant to obtain the first blend,
- obtaining a granulation solution comprising a solvent, one or more pharmaceutically acceptable excipient and/or at least one anti-oxidant,
- granulationg said blend with said granulation solution,
- drying the granulates, and
- mixing one or more pharmaceutically acceptable excipient to obtain second blend
- mixing first and second blend,
- compressing the resultant mixture into tablets
- and optionally coating said tablets.

The invention is illustrated by the following non-limiting examples:

### Comparative Example 1:

### Formulation I - Ivabradine oxalate formulation without antioxidant agents

**Table 1**

| **Ingredient** | **Amount (%)** |
|---|---|
| Ivabradine oxalate | 4-8 |
| Lactose | 60-75 |
| Pregelatinized starch | 6-15 |
| Maltodextrin | 6-15 |
| Magnesium stearate | 0,01-1,5 |
| Coating material | 2-4 |

The pharmaceutical formulation mentioned above is prepared as follows;
Ivabradine oxalate, lactose, pregelatinized starch and maltodextrin are mixed in a container in order to obtain a blend. The blend is then mixed with magnesium stearate and then compressed so as to form tablet. Tablets are then coated with coating solution in order to obtain film coated tablets.

### Example 2:

### Formulation II - Ivabradine oxalate formulation with antioxidant agents

**Table 2**

| **Ingredient** | **Amount (%)** |
|---|---|
| Ivabradine oxalate | 4-8 |
| Lactose | 60-75 |
| Pregelatinized starch | 6-15 |
| Maltodextrin | 6-15 |
| Ascorbic acid | 0,05-3 |
| Butyl hydroxytoluen | 0,05-3 |
| Crospovidone | 2-6 |
| Magnesium stearate | 0,01-1,5 |
| Coating material | 2-4 |

The pharmaceutical formulation mentioned above is prepared as follows;

Ivabradine oxalate, lactose, pregelatinized starch, maltodextrin and ascorbic acid are mixed in a container and granulated with the granulation solution comprising butyl hydroxytoluen. Granules are dried and sieved respectively. Obtained dried granules are mixed with crospovidone, magnesium stearate and then compressed as to form tablet. Tablets are then coated with coating solution in order to obtain film coated tablets.

### Stability tests

The above mentioned compressed film coated tablets of Example 1 and 2 were packed in blisters. These products in blisters were charged for their stability at 40°C - 75% relative humidity and observed for 6 months. After this 6 months period, the tablets were analyzed for impurity F and other unknown impurities by using HPLC.

Stability test results are as given below:

| **Impurity name** | **Initial** *(% by weight of ivabradine)* | | **After storage at 40°C / 75%RH for 6 months** *(% by weight of ivabradine)* | |
|---|---|---|---|---|
| | **Ex 1** | **Ex 2** | **Ex 1** | **Ex 2** |
| **Impurity F** | <0.05 | <0.05 | 0,55 | 0,057 |
| **Unknown impurities** | <0.05 | <0.05 | 0,22 | 0,092 |
| **Total impurities** | <0.05 | <0.05 | 1,3 | 0,15 |

It is clearly seen from the above data that; Impurity F, unknown impurities and total impurities in Ex 2 is less than the impurities in Ex 1.

## Claims

1. A pharmaceutical formulation comprising ivabradine oxalate as an active agent, at least one antioxidant and one or more pharmaceutically acceptable excipients; wherein said at least one antioxidant comprises butylated hydroxy toluene and ascorbic acid and said at least one antioxidant is being present in an amount of from 0,05% to 5% by weight of the total composition.

2. The pharmaceutical formulation according to claim 1, wherein said pharmaceutical formulation is in tablet dosage form.

3. The pharmaceutical formulation according to claim 1, wherein said at least one antioxidant is being present in an amount of from 0,1% to 2,50% by weight of the total composition.

4. The pharmaceutical formulation according to claim 1, wherein the weight ratio of antioxidant to ivabradine oxalate ranges from 0,008 to 0,75, preferably 0,03 to 0,4.

5. The pharmaceutical formulation according to claim 1, wherein the weight ratio of ascorbic acid to butylated hydroxytoluene ranges from 0,05 to 20, preferably 0,1 to 10 and more preferably 0,5 to 5.

6. The pharmaceutical formulation according to claim 1; wherein total impurities within said pharmaceutical formulation has increased not more than 1,0%.

7. A process for preparation of a pharmaceutical formulation as claimed in claim 1, comprising the steps of:
a. granulating ivabradine oxalate with the pharmaceutically acceptable excipients and/or at least one antioxidants for obtaining granulates,
b. drying said granulates
c. compressing said granulates into tablets, and
d. optionally coating said tablets.

8. The process according to claim 7;
wherein ivabraradine is granulated with a granulation solution which comprises a solvent, one or more pharmaceutically excipients and/or at least one antioxidant.

9. The process according to claim 8;
wherein ivabradine is granulated with a granulation solution which comprises ethanol and butylated hydroxytoluen.

## Patentansprüche

1. Pharmazeutische Formulierung aufweisend Ivabradin Oxalat als Wirkstoffe, mindestens ein Antioxidationsmittel und einen oder mehrere pharmazeutisch annehmbare Hilfsstoffe; wobei das mindestens eine Antioxidationsmittel aufweist: Butylhydroxytoluol und das mindestens eine Oxidationsmittel in einer Menge von 0,05 Gew.-% bis 5 Gew.-% der Gesamtzusammensetzung vorliegt.

2. Pharmazeutische Formulierung nach Anspruch 1, wobei die pharmazeutische Formulierung in Tablettenform vorliegt.

3. Pharmazeutische Formulierung nach Anspruch 1, wobei das mindestens eine Antioxidationsmittel in einer Menge von 0,1 Mass.-% bis 2,50 Mass.-% der Gesamtzusammensetzung vorliegt.

4. Pharmazeutische Formulierung nach Anspruch 1, wobei das Gewichtsverhältnis des Antioxidationsmittels zu Ivabradin Oxalat im Bereich von 0,008 bis 0,75, bevorzugt 0,03 bis 0,4 liegt.

5. Pharmazeutische Formulierung nach Anspruch 1, wobei das Gewichtsverhältnis der Ascorbinsäure zum Butylhydroxytoluol im Bereich von 0,05 bis 20, bevorzugt 0,1 bis 10 und weiter bevorzugt von 0,5 bis 5 liegt.

6. Pharmazeutische Formulierung nach Anspruch 1, wobei die Gesamtverunreinigung innerhalb der pharmazeutischen Formulierung nicht mehr als 1,0% zugenommen hat.

7. Verfahren zum Herstellen einer pharmazeutischen Formulierung nach Anspruch 1, aufweisend die Schritte:
a. Granulieren von Ivabradin Oxalat mit den pharmazeutisch annehmbaren Hilfsstoffen und/oder mindestens einem Antioxidationsmittel zum Erhalten von Granulaten,
b. Trocknen der Granulate,
c. Komprimieren der Granulate zu Tabletten,
d. optional Beschichten der Tabletten.

8. Verfahren nach Anspruch 7,
wobei Ivabradin mit einer Granulationslösung granuliert wird, welche ein Lösungsmittel, eine oder mehrere pharmazeutische Hilfsstoffe und/oder mindestens ein Antioxidationsmittel aufweist.

9. Verfahren nach Anspruch 8,
wobei Ivabradin mit einer Granulationslösung granuliert wird, welche Ethanol und Butylhydroxytoluol aufweist.

## Revendications

1. Formulation pharmaceutique comprenant de l'oxalate d'ivabradine en tant que principe actif, au moins un anti-oxydant et un ou plusieurs excipients pharmaceutiquement acceptables, ledit au moins un anti-oxydant comprenant de l'hydroxytoluène butylé et de l'acide ascorbique et ledit au moins un anti-oxydant étant présent dans une quantité de 0,05 % à 5 % en poids de la composition totale.

2. Formulation pharmaceutique selon la revendication 1, dans laquelle ladite formulation pharmaceutique est dans une forme posologique de comprimé.

3. Formulation pharmaceutique selon la revendication 1, dans laquelle ledit au moins un anti-oxydant est présent dans une quantité de 0,1 % à 2,50 % en poids de la composition totale.

4. Formulation pharmaceutique selon la revendication 1, dans laquelle le rapport de poids de l'anti-oxydant à l'oxalate d'ivabradine se situe dans la plage de 0,008 à 0,75, de préférence de 0,03 à 0,4.

5. Formulation pharmaceutique selon la revendication 1, dans laquelle le rapport de poids de l'acide ascorbique à l'hydroxytoluène butylé se situe dans la plage de 0,05 à 20, de préférence de 0,1 à 10 et, de manière davantage préférée, de 0,5 à 5.

6. Formulation pharmaceutique selon la revendication 1, dans laquelle les impuretés totales dans ladite formulation pharmaceutique n'ont pas augmenté de plus de 1,0 %.

7. Procédé pour la préparation d'une formulation pharmaceutique selon la revendication 1, comprenant les étapes consistant à :
a. granuler de l'oxalate d'ivabradine avec les excipients pharmaceutiquement acceptables et/ou au moins un anti-oxydant pour obtenir des granulés,
b. sécher lesdits granulés
c. comprimer lesdits granulés en comprimés, et
d. facultativement, enrober lesdits comprimés.

8. Procédé selon la revendication 7,
dans lequel l'ivabradine est granulée avec une solution de granulation qui comprend un solvant, un ou plusieurs excipients pharmaceutiquement acceptables et/ou au moins un anti-oxydant.

9. Procédé selon la revendication 8,
dans lequel l'ivabradine est granulée avec une solution de granulation qui comprend de l'éthanol et de l'hydroxytoluène butylé.
